# EUROPEAN PATENT APPLICATION

(11) **EP 0 526 706 A2**
(43) Date of publication of application: **10.02.1993**
(21) Application number: 92109176.5
(22) Date of filing: 01.06.1992
(51) Int. Cl.: B65D 83/06, B65D 77/04, A47G 19/24

(54) **Shaker device for dispensing powdered substances**

(30) Priority: 05.08.1991 US 740270
(71) Applicant: AMERICAN CYANAMID COMPANY, Wayne New Jersey 07470 (US)
(72) Inventor: Lanzilotti, Michael G., Spring Valley, New York 10977 (US); Boulay, William, Blauvelt, New York 10913 (US)
(74) Representative: Wächtershäuser, Günter, Prof. Dr.

(57) **Abstract**

An apparatus for dispensing solid substances, particularly medicaments, in the form of powders, granules, pellets, microspheres and the like comprising;
a) An inner container for the medicament having a top with a plurality of openings or a screen, and b) an outer housing encompassing the inner container having a top or cap with one or more apertures therein; the outer housing being large enough to allow the inner container to freely slide back and forth within the outer housing when the device is shaken toward the administration site thereby dislodging any aggregated medicament and dispersing it to administration site.

## Description

### FIELD OF THE INVENTION

This invention relates to dispensing devices for powdered substances and the like. More particularly, this invention relates to apparatus capable of dispensing solid substances such as materials in the form of powders, granules, pellets, micro- spheres, microshapes and the like. Such devices are suitable for the topical administration of powdered pharmaceuticals to humans or warm-blooded animals.

### BACKGROUND OF THE INVENTION

It is often desirable to dispense solids such as powders to a surface in the form of a dusting powder, and various devices are known for such uses. As illustrative but non-limiting examples, such devices can handle sugar, flour, corn starch and salt as well as chemicals, pharmaceuticals and so forth. In the pharmaceutical and veterinary area, it is known that dusting powders are useful to cover, protect and treat epithelial surfaces, ulcers and wounds. Often the dusting powders contain inert and insoluble substances which are finely subdivided such as talc, which absorb moisture and therefore act as cutaneous dessicants. The absorption of skin moisture decreases friction and discourages irritation and bacterial growth. Sometimes such dusting powders are used to deliver various biologically active substances such as antibiotics, antiseptics, antifungal agents and the like.

For most of these uses, the solid substances which would be dispersed as such dusting powders are usually in a fine state of subdivision. The powders or granules may be dispersed as very fine powders or they may be micronized or formulated into small micro-spheres or microshapes. In any case, the extemporaneously prepared powders are commonly dispersed in sifter-top packages consisting of a container having a top with a plurality of fine holes or apertures in it or the top may have a screen or sieve incorporated therein for dispersing the powder on the surface. Examples of such a dispensing device are the ordinary talcum powder container or the common container for dispensing confectionary sugar.

One of the problems often encountered with the delivery of powders in such a manner is that many powders exhibit poor flow properties. Many finely divided powders, microspheres or microparticles exhibit static charges when shaken which causes the powder to aggregate into clumps or adhere to the container surfaces inhibiting the flow of the powder through the apertures in the delivery device and onto the target surface. Such finely divided powders also absorb moisture from the air if not kept tightly sealed, further causing aggregation and poor flow. In a conventional sifter-top container for dispensing dusting powders, it is difficult to dispense the powder when it has aggregated in such a manner. The powder often sticks to the bottom of the container and does not flow to the delivery site unless the container is shaken vigorously or knocked against a hard surface whereupon the powder may become dislodged as a clump, preventing an even flow to the delivery site. Thus, there is a need for a device for dispensing powdered substances and the like which exhibit poor flow properties and which tend to aggregate, wherein the device provides a means of dislodging the aggregate powder and evenly dispensing the fine powder for delivery to the target site.

### OBJECTS AND SUMMARY OF THE INVENTION

It is an object of the invention to provide an improved dispensing apparatus for the delivery of solid substances such as powders, granules, pellets, microspheres, microshapes and the like.

It is a further object of the invention to provide an improved dispensing apparatus which is simple, easy to operate and which has a minimum of moving parts.

Yet another object of the present invention is to provide an improved dispensing device enabling improved feed of the solid substance for dispersal to the active site and which provides a means of dislodging aggregated substance to allow free flow to the delivery site. The invention provides a simple, uncomplicated means of dispensing micro- spheres, microshapes or powder which are statically charged or otherwise exhibit poor flow properties and which are difficult to deliver as a dusting powder through a sieve, screen or mesh of limited size.

A further object of the invention is to provide an improved dispensing device which is easily manufactured and which may be readily maintained in hygienic or sterile conditions.

A purpose of this device is to allow the free flow of solids in powdered form so they can be evenly dispersed to a targeted site.

A significant advantage of the present invention is its simplicity.

To achieve the above and other objects of the invention, there is provided an apparatus comprising an inner container for receiving and containing a solid substance, such as powders, granules, pellets, microspheres, microshapes and the like, said inner container having a top with a plurality of apertures for dispensing and dispersing the solid substance, and an outer housing or container encompassing the inner container in a manner allowing the inner container to slide back and forth within the outer housing and which outer housing has an opening adjacent to the top of the inner container through which the solid substance can flow. Upon shaking the entire apparatus back and forth, the solid substance contained within the inner container is dislodged and forced through the apertures on the top of the inner container and delivered to the desired site.

The size of the particles or powder suitable for use in the present invention are directly proportional to the size of the apertures in the top of the inner container. In this manner, the medicament is layered on the targeted area and uniformly dispersed. In addition, the aperture size to particle size relationship is important so that the apertures in the inner container are not obstructed by the powder.

As a feature of the above invention, the top of the inner container may have a screen incorporated therein which forms the apertures for dispensing and dispersing the solid particles.

The above invention is primarily suited for the delivery of solid pharmaceuticals in the form of finely divided powders, microspheres or microshapes, wherein the solid medicaments are administered topically as dusting powders to the treatment area.

The above and other objects, features and advantages of the invention will be found in the detailed description which follows hereinafter and which is illustrated in the accompanying drawings.

### BRIEF DESCRIPTION OF THE DRAWINGS

In the drawings:
FIG. 1 is a perspective side view showing the assembled dispensing device of the invention including the flowable solid substances contained therein;
FIG. 2 is a top view of the assembled device of FIG. 1;
FIG. 3 diagrammatically illustrates the operation of the invention;
FIG. 4 is an exploded view showing the component parts of the present invention;
FIG. 5 is a side view of the assembled device of an alternate design of the present invention wherein the outer housing has a threaded top;
FIG. 6 is an exploded view showing the component parts of an alternative design of the present invention wherein the outside container has a top with a hole in the middle and the bottom is a plug which is removable and replaceable.

### DETAILED DESCRIPTION

As has been generalized above, the apparatus of the invention comprises an inner container for receiving and containing a flowable solid substance such as a powder or granule, wherein the inner container has a top with a plurality of apertures for dispensing and dispersing the solid substance when inverted, and an outer housing into which the inner container is placed so that the top of the inner container is adjacent to the top of the outer housing. Further, the top of the outer housing has an opening through which the solid substance is allowed to flow. The top of the outer housing also has a means for retaining the inner container in its interior while allowing the inner container to slide freely back and forth in the interior of the outer housing. Upon inversion and shaking of the assembled device, the inner container slides back and forth within the outer housing or container and when the inner container is propelled against the retaining means in the top of the outer housing, it dislodges and ejects the powdered substances through the apertures in the top of the inner container, through the opening in the outer housing and onto the targeted area.

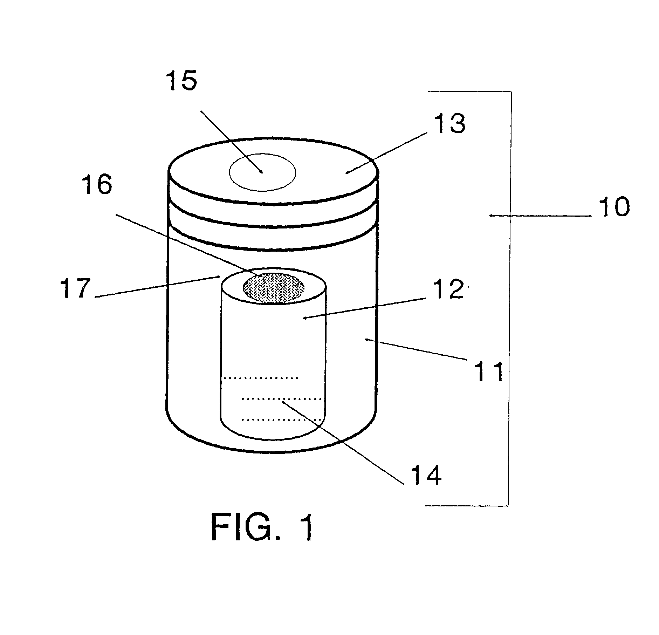

FIG. 1 shows a perspective side view of the assembled device 10 which is three main components, an outer housing 11, an inner container 12 and a cover or cap 13 to the outer housing 11. The inner container 12 contains the medicament 14 in the form of powders, granules, pellets, micro- spheres or microshapes and the like. The inner container 12 has a diameter slightly smaller than that of the outer housing 11 so that it fits in the outer housing in a centered relationship with respect to the outer housing during shaking. The inner container 12 has a top or retaining ring 17, also shown in FIG. 1 which holds a mesh or screen covering 16 to the top of the inner container 12 and fixed thereto. However, a plurality of small holes in place of the mesh screen in the top of the container would serve the same purpose and would be equally suitable. The cover or cap 13 to the outer housing has a hole 15 which is large enough to allow the medicament 14 to flow through but small enough so the inner container 12 is not ejected when the entire device is shaken. The top of the inner container 12 has a plurality of holes, preferred is a mesh or screen 16 whose apertures allow for the free flow of the powder to be evenly shaken to the targeted site. Holding the mesh or screen to the top of the inner container 12 is a retaining ring 17.

The dimensions of the apertures are subject to variation as determined by a variety of considerations. These considerations include: the nature of the material to be dispersed; the rate at which the material is preferably to be dispersed and the degree of dispersal of the material desired and so forth.

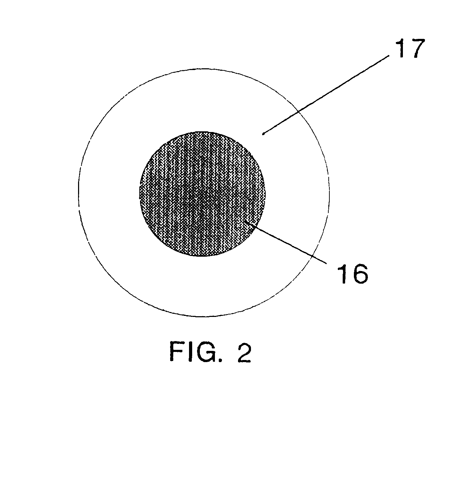

FIG. 2 is a top view of the inner container 12 which shows the mesh or screen 16 through which the medicament is shaken. Additionally, the top portion of the retaining ring 17 is shown.

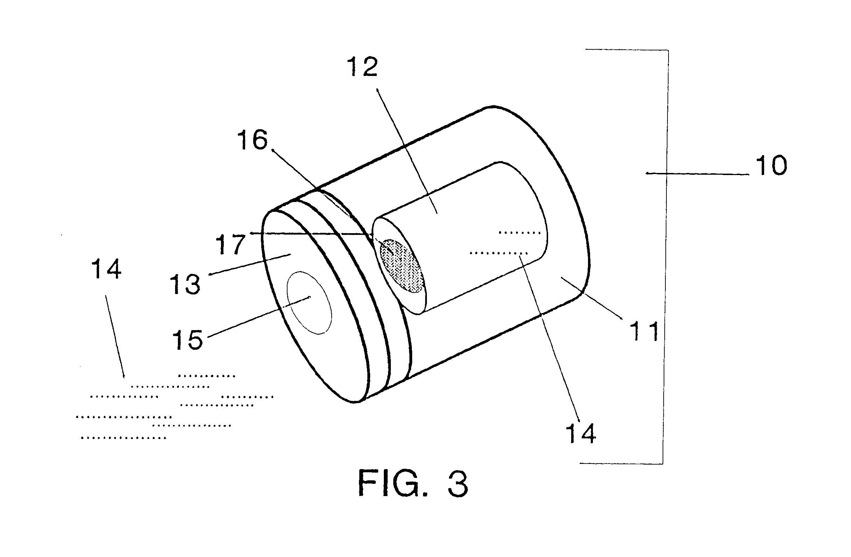

FIG. 3 diagrammatically illustrates the operation of the invention. The device 10 is shaken back and forth toward the target site so that the medicament 14 can be dispersed. While the device 10 is being operated, the inner container 12 containing the medicament 14 slides back and forth within the outer housing 11. The medicament in the form of a powder or microspheres is ejected through the screen or mesh 16 held to the inner container 12 with a retaining ring 17, through the hole 15 in the top of the outer container 11 toward the targeted site. In the device shown in Fig. 3, the opening 15 in the outer housing 11 is smaller than the top of the inner container 12 so that the inner container is retained in the interior of the outer housing and not ejected when the device is shaken.

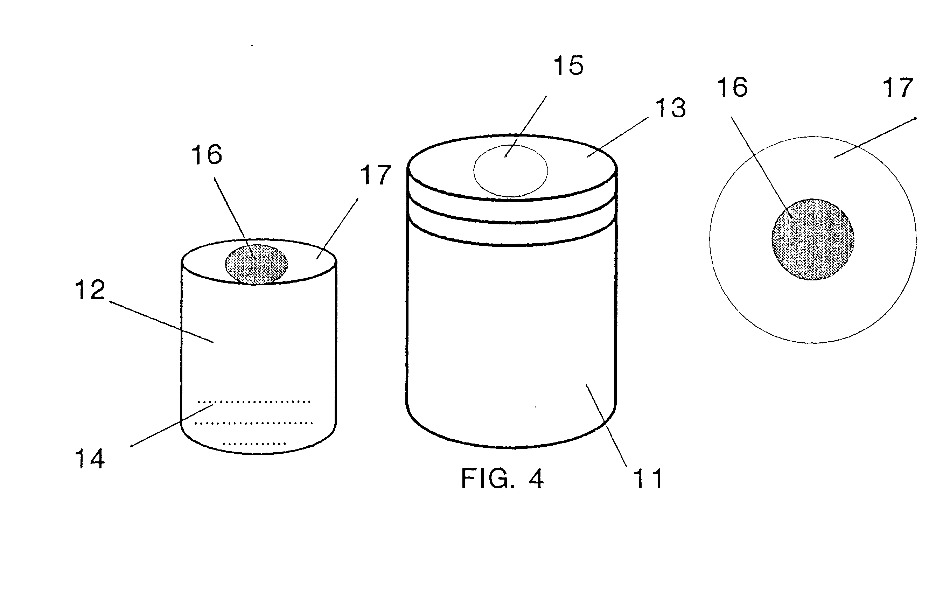

FIG. 4 illustrates each of the component parts of the device showing the outer housing 11 having a cover or cap 13 with a hole 15 in the middle, the inner container 12 which contains the medicament 14 and the retaining ring or cap 17 which holds the screen or mesh 16 to the top of the container.

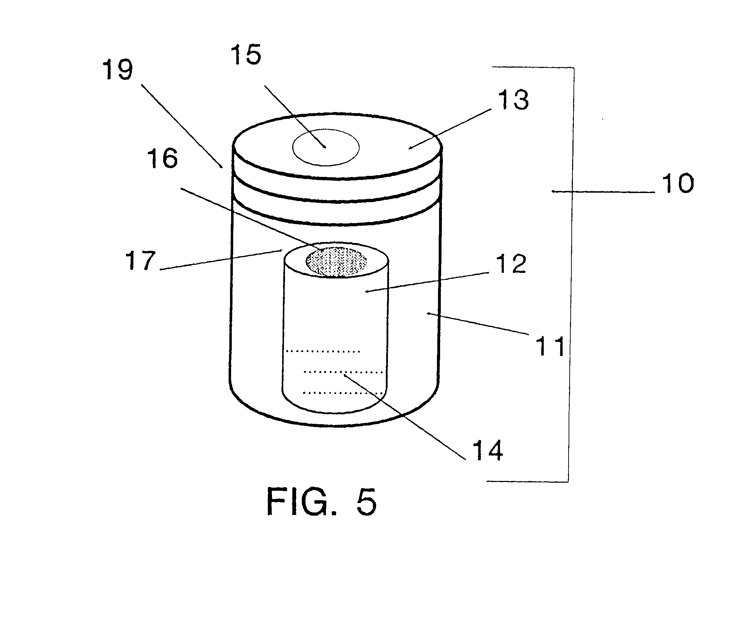

FIG. 5 is a side view of the fully assembled device 10 wherein the top 13 of the outer housing 11 is attached using a screw thread assembly 19.

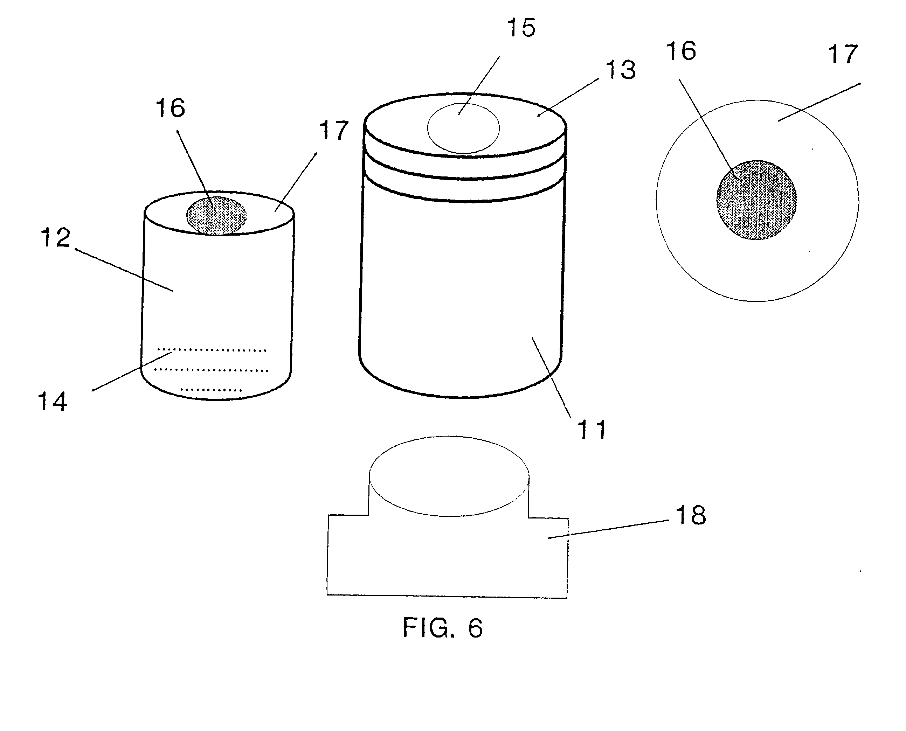

FIG. 6 illustrates each of the component parts of an alternate design of the device wherein the bottom of the outer housing 11 is replaceable with a bottom or plug 18 which is inserted after the insertion of the inner container 12. The plug 18 may be removable. In this manner the outer housing may be opened and the inner container may be replaced or refilled when the medicament is used up and the outer housing reused.

The device of the present invention is suitable for the topical administration of a variety of pharmaceutical substances such as antibiotics, antifungals, protectants, wound dressings and the like. In a preferred embodiment, the device is particularly useful for the topical delivery of tetracycline compounds, particularly minocycline, (7-dimethylamino-6-deoxy-6-demethyltetracycline) the preparation of which is described in U.S. Patents 3,148,212, 3,200,149 and 3,226,436. Preferably, the minocycline is in the form of microcapsules, microparticles or microspheres prepared by any of several techniques known in the art of microencapsula- tion including phase separation, coacervation, solvent evaporation, spray drying and the like. Preferably, the minocycline is administered as a sustained release composition for local administration consisting of a plurality of dry, discrete microparticles where the minocycline is dispersed in a matrix of a biocompatible and biodegradable polymer. One such composition may be used for local administration in the treatment of periodontal disease. In accordance with this method, the minocycline is administered as a dry powder which absorbs water upon administration and becomes tacky. This promotes adhesion to the tissues to be treated. The device of the present invention is particularly suited for the topical administration of this minocycline composition due to the nature of the microparticles which cause them to aggregate and exhibit poor flow properties. The device facilitates the delivery of the microparticles because the shaking motion dislodges the particles and disperses them evenly to the administration site.

Preferably, the device of the present invention should be constructed with medically sterilizable material such as shatterproof glass, plastic, metal or ceramic material so that the device can be filled with sterile medicament and used in the sterile field during surgery. Suitably, the inner container and outer housing are constructed with shatterproof glass and the top and screen are constructed of sterilizable metal.

It is also understood, of course, that while the form of the invention herein shown and described constitutes a preferred embodiment of the invention, it is not intended to illustrate all possible forms thereof. It will also be understood that the words used are words of description rather than limitation and various changes may be made without departing from the spirit and scope of the invention defined by the following claims.

## Claims

1. A device for the topical administration of medicaments in the form of powder, microspheres, or microparticles, said device comprising:
a) an inner container for containing the medicament having a top with a plurality of openings;
b) an outer housing encompassing the inner container and having a top or cap with one or more apertures therein; the outer housing being large enough to allow the inner container to freely slide back and forth within the outer housing when the entire device is shaken toward the administration site thereby dislodging any aggregated medicament and dispersing it to the administration site.

2. A device of Claim 1 wherein the plurality of openings is in the form of a screen or mesh.

3. A device of Claim 1 which is constructed of medically acceptable sterilizable material.

4. A device of Claim 1 wherein the top or cap of the outer housing is removable.

5. A device of Claim 1 wherein the size of the plurality of the openings in the top of the inner container is directly proportional to the size of the particles of medicament such that the medicament is evenly dispersed to the administration site.

6. A device of claim 1 wherein the medicament is selected from the group consisting of an antibiotic, an antifungal, a protectant or a wound dressing.

7. A device of Claim 6 wherein the medicament is minocycline.

8. A device of Claim 7 wherein the minocycline is in the form of microcapsules, microparticles or microspheres.

9. A device of Claim 6 wherein the medicament is in the form of microcapsules, microparticles or microspheres.

10. A device for the topical administration of medicaments in the form of powder, microspheres, or microparticles, said device comprising:
a) an inner container for containing the medicament having a top with a plurality of openings therein;
b) an outer housing encompassing the inner container and having a top or cap and a removable bottom, the top or cap having one or more apertures therein;
c) wherein the outer housing is large enough to allow the inner container to freely slide back and forth within the outer housing when the entire device is shaken toward the administration site thereby dislodging any aggregated medicament and dispersing it to the administration site.
